# EUROPEAN PATENT SPECIFICATION

(11) **EP 2 582 687 B1**
(45) Date of publication and mention of the grant of the patent: **12.11.2014**
(21) Application number: 11723932.7
(22) Date of filing: 31.05.2011
(51) Int. Cl.: C07D 317/34

(54) **2-oxo-1,3-dioxolane-4-carboxylic acid and derivatives thereof, their preparation and use**
2-Oxo-1,3-dioxolan-4-carbonsäure und ihre Derivate, ihre Herstellung und Verwendung
Acide 2-oxo-1,3-dioxolane-4-carboxylique et ses dérivés, sa préparation et utilisation associée

(30) Priority: 17.06.2010 EP 10166244
(43) Date of publication of application: 24.04.2013
(73) Proprietor: Construction Research & Technology GmbH, 83308 Trostberg (DE)
(72) Inventor: MECFEL-MARCZEWSKI, Joanna, 67117 Limburgerhof (DE); WALTHER, Burkhard, 84518 Garching (DE); MEZGER, Jochen, 67308 Lautersheim (DE); KIERAT, Radoslaw, 83352 Altenmarkt (DE); STAUDHAMER, Rosita, 84539 Ampfing (DE)
(86) International application number: PCT/EP2011/058945
(87) International publication number: WO 2011/157551

(56) References cited:
- EP-A1- 0 001 088
- WO-A1-2004/003001
- JP-A- 7 285 960
- JP-A- 2006 003 433
- TOMITA H ET AL: "MODEL REACTION FOR THE SYNTHESIS OF POLYHYDROXYURETHANES FROM CYCLIC CARBONATES WITH AMINES: SUBSTITUENT EFFECT ON THE REACTIVITY AND SELECTIVITY OF RING-OPENING DIRECTION IN THE REACTION OF FIVE-MEMBERED CYCLIC CARBONATES WITH AMINE", JOURNAL OF POLYMER SCIENCE, INTERSCIENCE PUBLISHERS, XX, vol. 39, no. 21, 1 January 2001 (2001-01-01), pages 3678-3685, XP001080699, cited in the application

## Description

The present invention relates to 2-Oxo-1,3-dioxolane-4-carboxylic acid and derivatives thereof, according to the general formula (V) 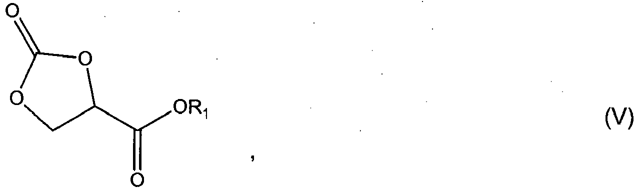 in which R₁ represents a negative charge, hydrogen or can be preferably Me or Et or an n-valent radical, which may be substituted with at most n-1 further 2-oxo-1,3-dioxolane-4-carboxyl groups, as well as a process for their preparation by means of carboxylation of the corresponding epoxides, a process for their transesterification and their use for the preparation of hydroxyurethanes and as end groups for the blocking of amines.

Structurally similar compounds are already known in the prior art. For example, WO2004/003001 A1 describes compounds of the general formula (I) 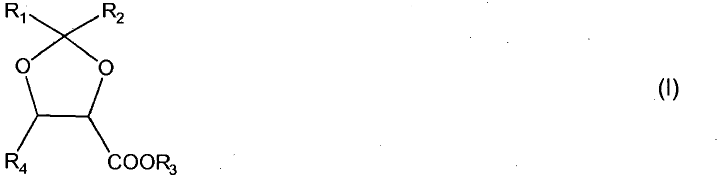 where R₁ and R₂ may be radicals independent of one another, R₁+R₂ = O or CR₁+R₂ may be a 3-6-membered cycloalkyl group. R₄ may be hydrogen, straight-chain or branched C₁₋₈-alkyl, C₅₋₁₂-cycloalkyl or C₆₋₁₅-aryl. R₃ may be straight-chain or branched C₁₋₈-alkyl or C₆₋₁₅-aryl. In general, WO2004/003001 describes the enzymatic racemate separation of the enantiomers of type (I) but without indicating a synthesis for these compounds.

EP 1941946 A1 describes the use of a carbonitride catalyst inter alia for the preparation of certain disubstituted organic carbonates. These may also be compounds of the general formula (II), 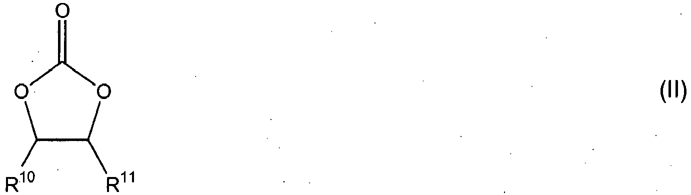 where R¹⁰ and R¹¹, independently of one another, are selected optional substituents. Possible meanings of the substituents are alkyl, aryl, heteroaryl and ester groups CO₂A, where A may in turn be alkyl or aryl, e.g. straight-chain or branched C₁₋₆-alkyl, preferably C₁₋₃-alkyl and particularly preferably methyl or ethyl. However, no syntheses for 2-oxo-1,3-dioxolane systems are stated.

JP 2006-003433 A discloses a sealing composition for liquid crystal display elements which comprises a compound of the general formula (III), 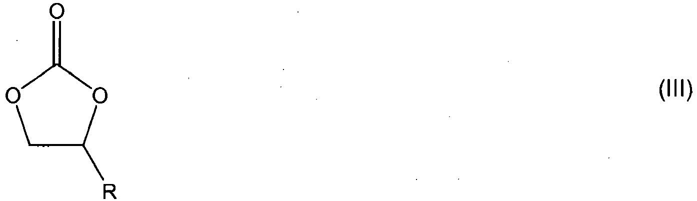 where R is H, a hydroxyl group, a cyano group, a carboxylic acid group, an optionally substituted aromatic ring, a straight-chain, branched or cyclic alkyl group, an acyl group or an ester group. However, it is not stated in what direction the ester group points and which further radical it carries. Neither is any specific synthesis for these 2-oxo-1,3-dioxolane systems stated.

EP 0001088 A1 describes inter alia 2-oxo-1,3-dioxolanes of the general formula (IV), where R can be H or CH₃.

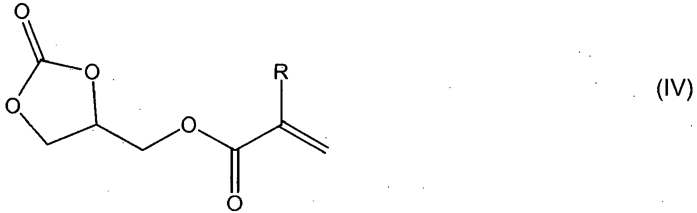

Polyurethanes based on polyisocyanates belong to the prior art. These are used for example as adhesives, sealants, casting compositions, as corrosion protection and for coatings. The high resistance to acid, alkalis and chemicals of the cured compositions obtained in this way are advantageous. However, monomeric low molecular weight (poly)isocyanate compounds are toxicologically unacceptable, especially if they are readily volatile or migrate.

Polyurethane systems can also be obtained starting from cyclic carbonate compounds which are toxicologically acceptable. Thus, glycerol carbonate (4-(hydroxymethyl)-2-oxo-1,3-dioxolane) is used in cosmetics, for example. Cyclic carbonate compounds react with amines to give hydroxyurethanes.

However, simple cyclic carbonates such as e.g. 4-methyl-2-oxo-1,3-dioxolane or said 4-(hydroxymethyl)-2-oxo-1,3-dioxolane are not particularly reactive. Studies have been carried out, cf. H. Tomita, F. Sanda, T. Endo, Journal of Polymer Science: Part A: Polymer Chemistry, Vol. 39, 3678-3685 (2001), according to which the reactivity of the 2-oxo-1,3-dioxolanes substituted in 4-position by the group R with amines increases in the order: R = Me < R = H < R = Ph < R = CH₂OPh « R = CF₃. Unfortunately, such fluorinated compounds are not readily accessible, expensive and (e.g. in the event of fire) potentially toxic. Moreover, low molecular weight monomeric 2-oxo-1,3-dioxolanes are not suitable as binders. Rather, reactive functional groups for example in 4-position are required in order to prepare relatively high molecular weight multifunctional binders which can then be reacted with amines for the polyurethane formation. The industrial accessibility of these 2-oxo-1,3-dioxolanes also plays an important role.

It was the object of the present invention to essentially avoid at least some of the disadvantages of the prior art described above. In particular, the aim was to provide a 2-oxo-1,3-dioxolane system which is acceptable, readily accessible and highly reactive with amines and carries at least one further reactive functional group.

This object has been achieved with the features of the independent claims. The dependent claims relate to preferred embodiments.

The present invention provides 2-Oxo-1,3-dioxolane-4-carboxylic acid, or a derivative thereof, according to the general formula (V), 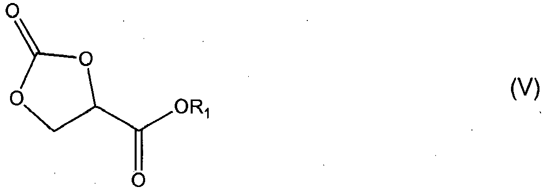 wherein R₁ represents a negative charge, hydrogen, or a group selected from straight-chain or branched aliphatic groups, aryl groups, aralkyl groups and alkylaryl groups.

In case R₁ represents a negative charge the counterion to compensate that charge is preferably selected from alkali metal and alkaline earth metal cations, preferrably from Li⁺, Na⁺, K⁺, and ½ Ca²⁺. The subject derivative is thus an alkali metal or alkaline earth metal salt. In case R₁ represents hydrogen, the subject compound is thus 2-Oxo-1,3-dioxolane-4-carboxylic acid.

In case R₁ represents a group selected from straight-chain or branched aliphatic groups, aryl groups, aralkyl groups and alkylaryl groups the said derivative is thus an ester. In particular R₁ represents a C₁₋₁₂-alkyl group.

R₁ may, for example, be selected from methyl, ethyl, n-propyl, isopropyl, n-butyl, sec-butyl, tert-butyl, n-pentyl, neopentyl, n-hexyl, 2-ethyl-n-hexyl, n-lauryl, cyclohexyl, phenyl and benzyl.

In particular, the 2-oxo-1,3-dioxolane-4-carboxylic acid ester according to the invention is 4-methoxycarbonyl-2-oxo-1,3-dioxolane or 4-ethoxycarbonyl-2-oxo-1,3-dioxolane.

However, it is likewise possible that R₁ is an n-valent radical derived by abstraction of the OH groups of an n-valent polyol which may be substituted by at most n-1 further 2-oxo-1,3-dioxolane-4-carboxylate groups of the general formula (VI)

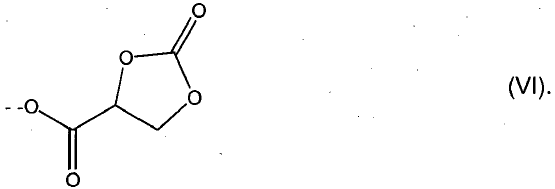

If fewer than n-1 further 2-oxo-1,3-dioxolane-4-carboxylate groups are present, R₁ is additionally substituted with the stoichiometrically required number of OH groups.

In these relatively high molecular weight 2-oxo-1,3-dioxolane-4-carboxylic acid esters, the n-valent polyol can comprise for example C₂₋₄-(poly)oxyalkylene groups, i.e. groups derived from ethylene oxide, propylene oxide and/or butylene oxide and having one or more oxyalkylene repeat units. Preferably, n = 2 to 5. Examples of such relatively high molecular weight multifunctional compounds suitable as binders are discussed below.

A further subject matter of the present invention is considered to be the preparation of the low molecular weight monomeric 2-oxo-1,3-dioxolane-4-carboxylic acid esters according to the invention. These 2-oxo-1,3-dioxolane-4-carboxylic acid esters can be prepared for example by reacting the corresponding epoxides of the formula (VII), where R₁ has the stated meaning, with CO₂.

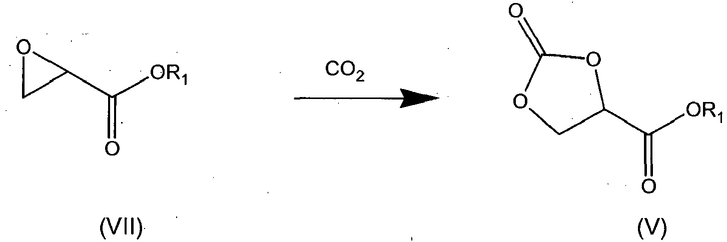

Epoxides of the formula (VII) are commercially available compounds well-known in the prior art which can be prepared for example by means of epoxidation of the corresponding acrylic acid esters (VIII); for R₁ = Me, cf. e.g. Organic Syntheses, Vol. 83, p. 162 (2006):

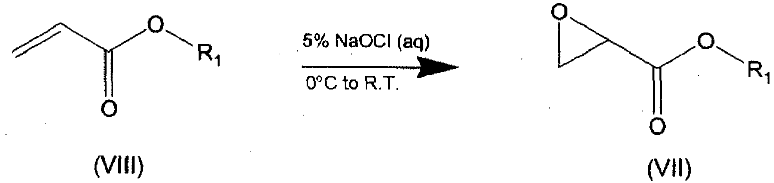

Alternative syntheses are also known; for R₁ = Et cf. e.g. Organic Syntheses, Coll. Vol. 10, p. 401 (2004); Vol. 75, p. 37 (1998).

The temperature of the aforementioned reaction with CO₂ can be varied within wide ranges. It should expediently be in the range from 15°C to 150°C, preferably in the range from 30°C to 100°C, and in particular in the range from 60°C to 80 °C. The reaction can be carried out in open apparatuses at ambient pressure (ca. 1 bar), for example by means of passing gaseous CO₂ through a suitable reaction solution. However, the reaction can also take place in closed systems at an increased pressure, for example at a pressure of from 1 bar to 100 bars, preferably from 20 bars to 100 bars, and in particular at about 80 bars.

The reaction with CO₂ can take place without a solvent since the starting materials and the products are generally liquid. However, it has proven to be expedient to carry out the reaction in polar aprotic solvents. A nonexhaustive list of suitable solvents includes tetrabutyl methyl ether, acetonitrile, acetone, tetrahydrofuran, dimethyl carbonate, toluene, xylene, N-methyl-2-pyrrolidone, N-ethyl-2-pyrrolidone, and mixtures thereof.

The reaction with CO₂ can generally be carried out without catalyst. However, the procedure expediently involves working in the presence of a catalyst which is selected from metal halides and halogen salts of organic nitrogen compounds, and mixtures thereof.

As has been established above, low molecular weight, monomeric 2-oxo-1,3-dioxolane-4-carboxylic acid esters are not suitable as binders. However, the COOR₁ group has the advantage that it is available for further reactions. Thus, relatively high molecular weight, multifunctional representatives of the compound of the general formula (V) according to the invention can be prepared by means of transesterification. Accordingly, a process for the preparation of these relatively high molecular weight multifunctional esters is judged to be a further subject matter of the present invention, where a low molecular weight, monomeric 2-oxo-1,3-dioxolane-4-carboxylic acid ester of the general formula (V) is transesterified with an n-valent polyol.

In said process, the transesterification is carried out in the presence of an enzymatic catalyst or an acidic cation exchanger. One of the difficulties which was associated with this transesterification reaction was to find catalysts which catalyse the transesterification at the -COOR₁ group, but do not lead to attacks on the -O-COO-group. The aforementioned catalysts circumvent these difficulties. Novozym^{®} 435 from Novozymes A/S, an immobilized lipase, and the H⁺ form of Amberlite^{®} 200 from Rohm & Haas Company, i.e. a strongly acidic cation exchanger, have proven to be particularly suitable.

The polyol and the low molecular weight 2-oxo-1,3-dioxolane-4-carboxylic acid ester are preferably used in stoichiometric fractions, where the conversion of the transesterification should preferably be above 80%, based on the low molecular weight 2-oxo-1,3-dioxolane-4-carboxylic acid ester used. The reaction temperature is in the range from 50 to 100°C, in which case Novozym^{®} 435 should be used at about 50 to 80°C and Amberlite^{®} 200 should be used at ca. 100°C. The transesterification with Novozym^{®} 435 can expediently be carried out without solvents; the transesterification with Amberlite^{®} 200 is expediently carried out in a suitable solvent. The reaction is expediently carried out until essentially the calculated amount of R₁-OH has been distilled off.

Suitable polyols are, for example, diols, in particular glycols, triols and tetraols, such as e.g. 1,4-butanediol, neopentyl glycol (2,2-dimethylolpropane), 1,1,1-trimethylolpropane, pentaerythritol and tetramethylolmethane. Said polyols can also be modified with C₂₋₄-alkylene oxides, in particular ethylene oxide and propylene oxide. In general, it is possible to use all polyols which can also be used for the preparation of conventional polyurethanes.

Alternatively, it is possible to firstly transesterify low molecular weight acrylic acid esters with said polyols, then to epoxidize them and then to carboxylate them with CO₂. This gives compounds which likewise fall under the general formula (V).

The invention further provides the use of the 2-oxo-1,3-dioxolane-4-carboxylic acid esters for the preparation of hydroxyurethanes. The cyclic carbonate compounds according to the invention react with amines to give hydroxyurethanes.

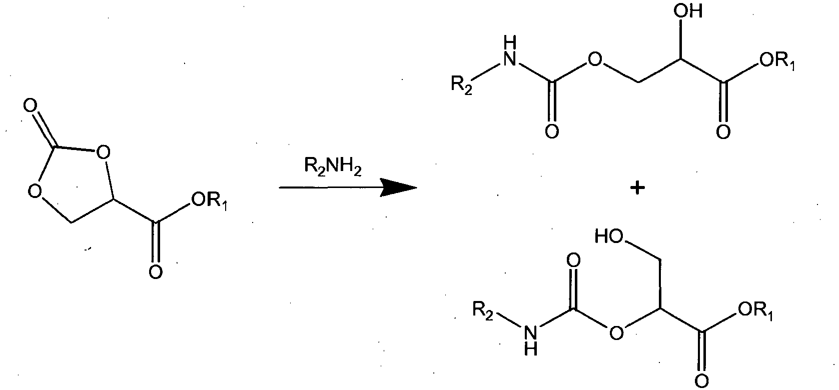

Here in principle two different hydroxyurethanes are possible, namely hydroxyurethanes with primary or secondary hydroxyl groups. In this respect, it has been shown that the electron-withdrawing COOR₁ group diverts the reaction essentially in the direction of the hydroxyurethanes with secondary hydroxyl groups since, in the event of attack of the nucleophilic nitrogen atom, the negative charge on the oxygen atom which is closer to the COOR₁ group is better stabilized. Hydroxyurethanes with secondary hydroxyl groups have the additional advantage that the back-reaction is hindered. Theoretically, an attack of the amine at the ester group would also be conceivable; however, it was shown analytically that the amine essentially attacks only the 2-oxo-1,3-dioxolane group.

Suitable amines are primary and secondary amines with alkyl groups, aryl groups, aralkyl groups, and alkylaryl groups as radicals. Primary amines react more quickly than secondary amines; aliphatic amines react more quickly than aromatic amines. As regards the relative reactivities of different amines, compare C. Diakoumakos, D. Kotzev, Non-Isocyanate-Based Polyurethanes Derived upon the Reaction of Amines with Cyclocarbonate Resins, Macromol. Symp., 216, 37-46 (2004), in particular scheme 4 on p. 45. All of the amines specified therein are also suitable for carrying out the present invention. Relatively high molecular weight amines such as e.g. Jeffamine^{®} from Huntsman Corp. and polyether amines from BASF SE are also suitable.

As is shown below by reference to examples, the 2-oxo-1,3-dioxolane-4-carboxylic acid esters according to the invention are significantly more reactive towards amines than for example the comparable compounds glycerol carbonate (4-(hydroxymethyl)-2-oxo-1,3-dioxolane) and propylene carbonate (4-methyl-2-oxo-1,3-dioxolane). This is true both for the low molecular weight representatives and also for the relatively high molecular weight representatives. The reactivity of the 4-methoxycarbonyl-2-oxo-1,3-dioxolane according to the invention towards amines must be in the order of magnitude of the 4-trifluoromethyl-2-oxo-1,3-dioxolane investigated in "H. Tomita, F. Sanda, T. Endo, Journal of Polymer Science: Part A: Polymer Chemistry, Vol. 39, 3678-3685 (2001)", but without having the disadvantages of the CF₃ group described above.

An additional advantage of the polyhydroxyurethane systems lies in the higher hydrophilicity of these systems, which can be attributed to the OH groups present. These OH groups are in principle also available for the crosslinking with polyisocyanates, although the isocyanate-free systems possible according to the invention are preferred on account of their lower toxicity.

Moreover, when producing polyhydroxyurethane systems which are based on 2-oxo-1,3-dioxolanes, bubble formation as a result of CO₂ that is formed may not arise, even in the presence of moisture. Consequently, largely pore- and bubble-free coatings are possible, which is sometimes problematic for classic polyurethane systems. Furthermore, the thermal stability of such polyhydroxyurethane systems is also higher than the stability of classic polyurethane systems.

Moreover, the low molecular weight 2-oxo-1,3-dioxolane-4-carboxylic acid esters can be used to block amines as end groups (so-called "end caps"), which constitutes a further subject matter of the present invention. This is also of interest with regard to conventional, amine-crosslinked polyurethane systems since an amine excess can lead to discolorations, while an isocyanate excess is toxicologically unacceptable.

The present invention is now illustrated in more detail using the examples below with reference to the attached drawings: These show:
- Fig. 1: the progress over time of the reaction of various 2-oxo-1,3-dioxolanes with ethanolamine,
- Fig. 2: the progress over time of the reaction of various 2-oxo-1,3-dioxolanes with benzylamine,
- Fig. 3: the progress over time of the reaction of various 2-oxo-1,3-dioxolanes with isophoronediamine,
- Fig. 4: the progress over time of the reaction of various 2-oxo-1,3-dioxolanes with Jeffamin^{®} D 400,
- Fig. 5: the progress over time of the reaction of various binders based on 2-oxo-1,3-dioxolanes with n-butylamine.

### Example 1a: Preparation of 4-methoxycarbonyl-2-oxo-1,3-dioxolane

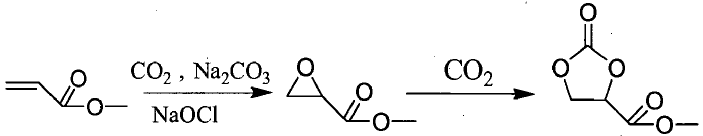

80 g of sodium carbonate were dissolved in 200 ml of distilled water in a 1000 ml three-neck flask. The solution was cooled to 10°C. 58.5 g of methyl acrylate were then added and, after ca. 10 minutes, likewise at 10°C, 400 ml of a 7% strength aqueous sodium hypochlorite solution were stirred in. Then, the system was immediately flushed intensively with CO₂. The temperature was allowed to increase to room temperature. The flask was flushed intensively with CO₂ for a further 1 h at ca. 25 to 30°C, during which the temperature was held in the stated range through occasional cooling with an ice bath. The resulting white solid was filtered off via a suction filter. The filtrate was extracted with 4 x 90 ml of dichloromethane. The combined organic phase was dried with sodium sulphate and filtered off. The filtrate was removed on a rotary evaporator. Methyl epoxypropionate was obtained in 50 to 60% yield and a purity of 97%.

20 g of the methyl epoxypropionate were mixed with 20 g of tert-butyl methyl ether and 1 g of tetrabutylammonium bromide. The homogeneous mixture was transferred to a 100 ml pressurized reactor and carboxylated for 4 days at 40°C and a CO₂ pressure of 20 bar. Following carboxylation, a two-phase system was obtained; the upper phase consisted of tert-butyl methyl ether, and the lower phase consisted of 4-methoxy-carbonyl-2-oxo-1,3-dioxolane (purity 94% (GC), yield 94%).

The product was characterized as follows: ¹H NMR (500 MHz, CDCl₃) δ: 3.82 (3H, s, CH₃), 4.50 (1 H, dd, J = 5.5, 9.0, CH₂), 4.66 (1 H, dd, J = 9.0, 9.0, CH₂), 5.09 (1 H, dd, J = 9.0, 5.5, CH); ¹³C NMR (125 MHz, CDCl₃) 6: 53.81 (CH₃), 67.00 (CH₂), 72.34 (CH), 153.97 (-O-CO-O-), 167.42 (-CO-O-); IR (neat): 1812 cm⁻¹, (-O-CO-O-), 1742 cm⁻¹ (-CO-O-).

### Example 1b: Preparation of 4-ethoxycarbonyl-2-oxo-1,3-dioxolane

Example 1 a was repeated as described hereinabove, with the exception that ethyl acrylate was used instead of methyl acrylate. The results were essentially as stated in example 1a, with the exception that 4-ethoxycarbonyl-2-oxo-1,3-dioxolane was obtained.

### Example 2: Preparation of 4-methoxycarbonyl-2-oxo-1,3-dioxolane

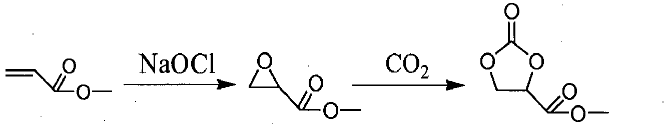

940 ml of a 7% strength aqueous sodium hypochlorite solution were introduced as initial charge in a 2000 ml three-neck flask. The solution was cooled to 0°C with the help of an ice/salt water bath. 58.5 g of methyl acrylate were then added and the mixture was held at 0°C for 30 minutes. The low-temperature mixture was then removed and further stirred for ca. 1.5 h such that the mixture heated up by itself (65-70°C). A colourless, cloudy solution was formed. Then, the solution was cooled to room temperature and extracted with 4 x 150 ml of dichloromethane. The combined organic phase was dried with magnesium sulphate and filtered off. The filtrate was removed on a rotary evaporator. Methyl epoxypropionate was obtained in 70 to 80% yield and a purity of 97%. The further reaction to the 4-methoxycarbonyl-2-oxo-1,3-dioxolane proceeded as described in Example 1. Example 3: Preparation of 4-methoxycarbonyl-2-oxo-1,3-dioxolane 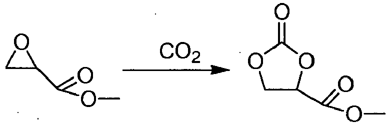 20 g of methyl epoxypropionate were mixed with 20 g of acetonitrile, 1.5 g of benzyltrimethylammonium chloride and 1.5 g of ZnBr₂. The homogeneous mixture was transferred to a 100 ml pressurized reactor and carboxylated for 6 days at 25°C and a CO₂ pressure of 30 bar. Following carboxylation, the mixture was diluted with 100 g of acetonitrile. The mixture was purified with aluminium oxide and activated carbon. Then, the acetonitrile was distilled off. This gave 4-methoxycarbonyl-2-oxo-1,3-dioxolane (purity 72% (GC), yield 65%).

### Example 4: Preparation of 4-methoxycarbonyl-2-oxo-1,3-dioxolane

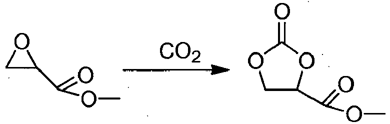

20 g of methyl epoxypropionate were mixed with 20 g of tert-butyl methyl ether, 1.5 g of tetrabutylammonium bromide and 1.5 g of potassium iodide. The homogeneous mixture was transferred to a 100 ml pressurized reactor and carboxylated for 6 days at 50 °C and a CO₂ pressure of 30 bar. Following the carboxylation, a two-phase system was obtained; the upper phase consisted of tert-butyl methyl ether, and the lower phase consisted of 4-methoxycarbonyl-2-oxo-1,3-dioxolane (purity 83% (GC), yield 79%).

### Example 5: Preparation of binder 1

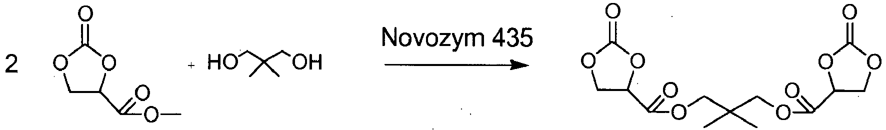

0.2 mol of 4-methoxycarbonyl-2-oxo-1,3-dioxolane ("GECA") were mixed with 0.1 mol of neopentyl glycol (Sigma-Aldrich). 5% by weight (based on GECA) of Novozym^{®} 435 (Novozymes A/S) were added thereto. The mixture was stirred and heated to 55 to 60°C. After 72 h, 0.2 mol of methanol had distilled off and the reaction was complete.

### Example 6: Preparation of binder 2

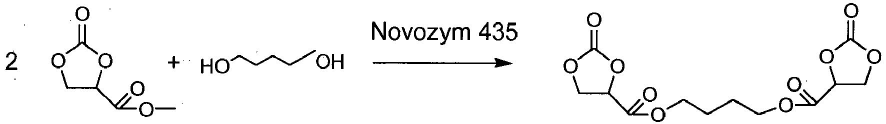

0.2 mol of 4-methoxycarbonyl-2-oxo-1,3-dioxolane ("GECA") were mixed with 0.1 mol of 1,4-butanediol (Sigma-Aldrich). 5% by weight (based on GECA) of Novozym^{®} 435 (Novozymes A/S) were added thereto. The mixture was stirred and heated to 55 to 60°C. After 72 h, 0.2 mol of methanol had distilled off and the reaction was complete.

### Example 7: Preparation of binder 3

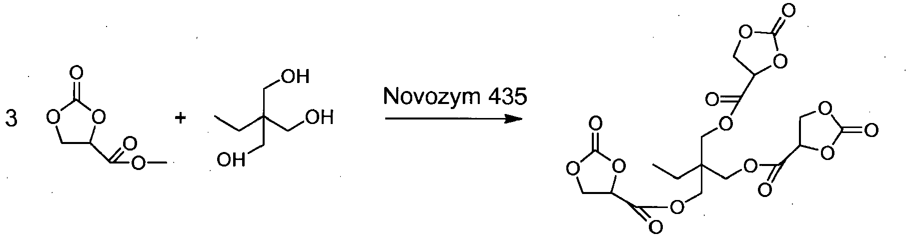

0.3 mol of 4-methoxycarbonyl-2-oxo-1,3-dioxolane ("GECA") were mixed with 0.1 mol of 1,1,1-trimethylolpropane (Sigma-Aldrich). 5% by weight (based on GECA) of Novozym^{®} 435 (Novozymes A/S) were added thereto. The mixture was stirred and heated to 55 to 60°C. After 72 h, 0.3 mol of methanol had distilled off and the reaction was complete.

### Example 8: Preparation of binder 4

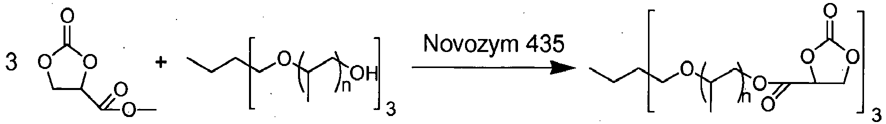

0.3 mol of 4-methoxycarbonyl-2-oxo-1,3-dioxolane ("GECA") were mixed with 0.1 mol of 1,1,1-trimethylolpropane propoxylate (Sigma-Aldrich, average molecular weight (Mₙ) ca. 308). 5% by weight (based on GECA) of Novozym^{®} 435 (Novozymes A/S) were added thereto. The mixture was stirred and heated to 55 to 60°C. After 72 h, 0.3 mol of methanol had distilled off and the reaction was complete.

### Example 9: Preparation of binder 1

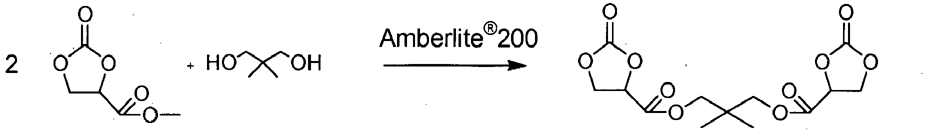

0.2 mol of 4-methoxycarbonyl-2-oxo-1,3-dioxolane ("GECA") were mixed with 0.1 mol of neopentyl glycol (Sigma-Aldrich) and 200 ml of cyclohexane. 5% by weight (based on GECA) of Amberlite^{®} 200 (Fluka) were added thereto. The mixture was stirred at 100°C on a water separator. After 5 hours, 0.2 mol of methanol had separated off and the reaction was complete.

### Example 10: Preparation of binder 3

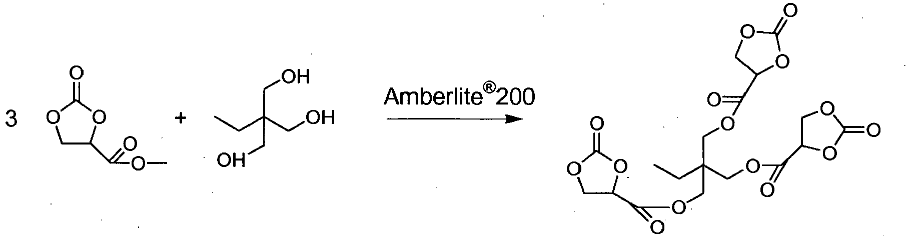

0.3 mol of 4-methoxycarbonyl-2-oxo-1,3-dioxolane ("GECA") were mixed with 0.1 mol of 1,1,1-trimethylolpropane (Sigma-Aldrich). 5% by weight (based on GECA) of Amberlite^{®} 200 (Fluka) were added thereto. The mixture was stirred at 100°C on a water separator. After 5 hours, 0.3 mol of methanol had separated off and the reaction was complete.

### Example 11: Reaction of 2-oxo-1,3-dioxolanes with ethanolamine

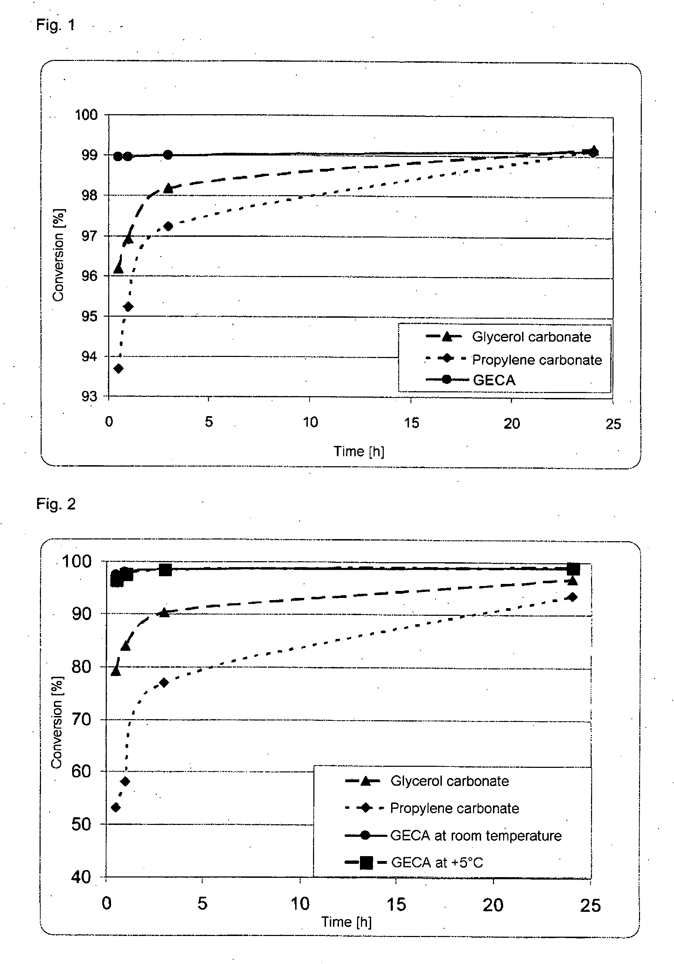

0.1 mol of ethanolamine were mixed with 0.1 mol of 4-methoxycarbonyl-2-oxo-1,3-dioxolane ("GECA") and stirred at room temperature. After 0.5 h, 1 h, 3 h and 24 h, the amine number was determined by means of titration and used to calculate the conversion. This procedure was also carried out with 4-methyl-2-oxo-1,3-dioxolane ("propylene carbonate") and 4-(hydroxymethyl)-2-oxo-1,3-dioxolane ("glycerol carbonate"). The results are reproduced graphically in Fig. 1 and show the high reactivity of 4-methoxycarbonyl-2-oxo-1,3-dioxolane.

### Example 12: Reaction of 2-oxo-1,3-dioxolanes with benzylamine

Example 11 was repeated using benzylamine instead of ethanolamine. 4-Methoxy-carbonyl-2-oxo-1,3-dioxolane ("GECA") was investigated both at room temperature and also at +5°C, whereas 4-methyl-2-oxo-1,3-dioxolane ("propylene carbonate") and 4-(hydroxymethyl)-2-oxo-1,3-dioxolane ("glycerol carbonate") were tested just at room temperature. The results are reproduced graphically in Fig. 2 and impressively show the exceptional reactivity of 4-methoxycarbonyl-2-oxo-1,3-dioxolane (even at +5°C).

### Example 13: Reaction of 2-oxo-1,3-dioxolanes with isophoronediamine

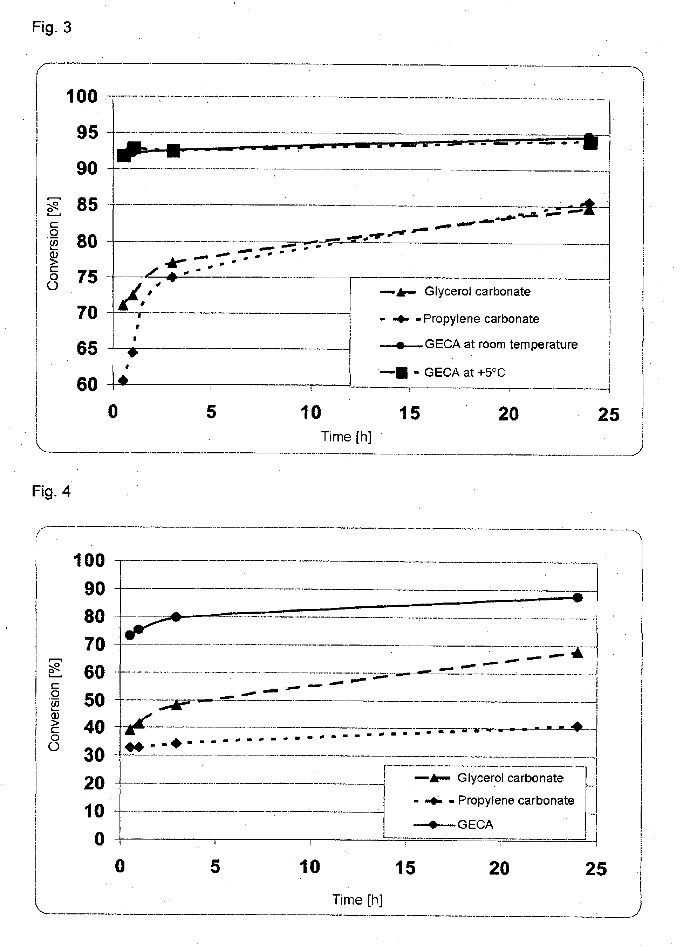

0.1 mol of isophoronediamine was mixed with 0.2 mol of 4-methoxycarbonyl-2-oxo-1,3-dioxolane ("GECA") and stirred at room temperature. After 0.5 h, 1 h, 3 h and 24 h, the amine number was determined by means of titration and used to calculate the conversion. This procedure was also carried out with 4-methoxycarbonyl-2-oxo-1,3-dioxolane ("GECA") at +5°C and with 4-methyl-2-oxo-1,3-dioxolane ("propylene carbonate") and 4-(hydroxymethyl)-2-oxo-1,3-dioxolane ("glycerol carbonate") at room temperature. The results are reproduced graphically in Fig. 3 and impressively show the exceptional reactivity of 4-methoxycarbonyl-2-oxo-1,3-dioxolane (even at +5°C).

### Example 14: Reaction of 2-oxo-1,3-dioxolanes with Jeffamin® D 400

Example 13 was repeated using Jeffamin^{®} D 400. Since the reaction with high molecular weight amines generally proceeded more slowly than with low molecular weight amines, the 4-methoxycarbonyl-2-oxo-1,3-dioxolane (GECA) was also only investigated at room temperature. The results are reproduced graphically in Fig. 4 and also show in the present case the high reactivity of 4-methoxycarbonyl-2-oxo-1,3-dioxolane.

### Example 15: Reaction of binder 3 compared to the prior art

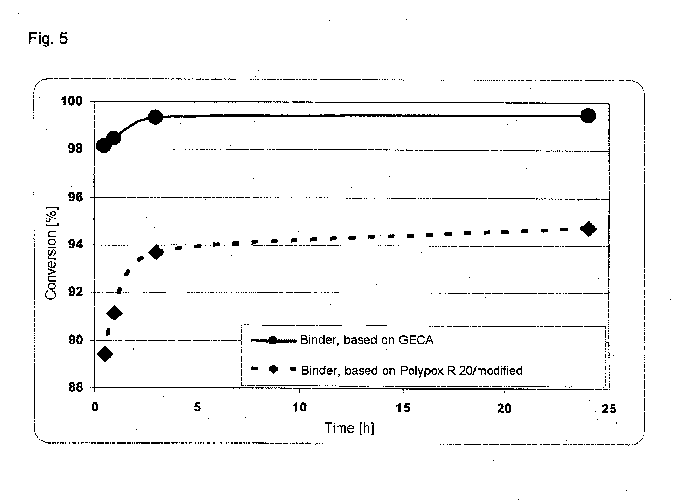

0.1 mol of binder 3 from example 7 or 10 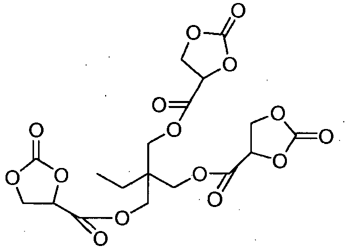 was mixed with 0.3 mol of n-butylamine and stirred at room temperature. After 0.5 h, 1 h, 3 h and 24 h, the amine number was determined by means of titration and used to calculate the conversion. This procedure was also carried out with carboxylated Polypox^{®} R20 (UPPC AG), a trifunctional epoxide which was carboxylated with CO₂ by ourselves, 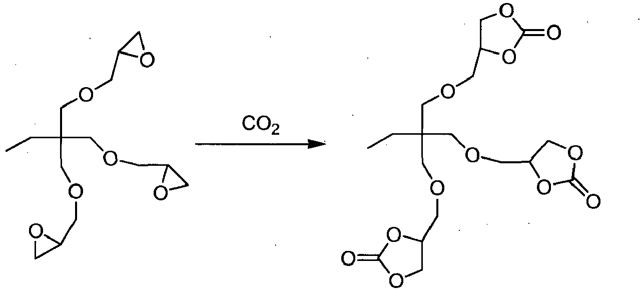 at room temperature. The results are reproduced graphically in Fig. 5 and show the exceptional reactivity of our binder.

### Example 16: Film formation with isophoronediamine

0.000666 mol of binder 3 from example 7 or 10 was mixed with 0.001 mol of isophoronediamine (binder in excess). The two components were stirred together by hand for 20 seconds, after which a film 300 µm in thickness was drawn (pot time 2 min, tack-free after 6 hours).

### Example 17: Film formation with 1,3-cyclohexanebis(methylamine)

0.000666 mol of binder 3 from example 7 or 10 was mixed with 0.001 mol of 1,3-cyclo-hexanebis(methylamine) (binder in excess). The two components were stirred together by hand for 20 seconds, after which a film 300 µm in thickness was drawn (pot time 2 min, tack-free after 7 hours).

## Claims

1. 2-Oxo-1,3-dioxolane-4-carboxylic acid ester of the general formula (V), 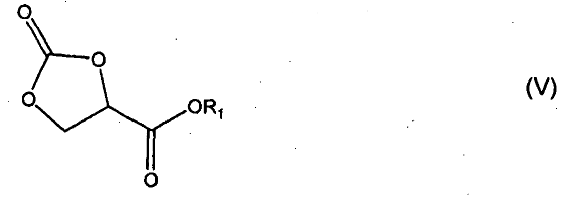 wherein R₁ is selected from methyl, ethyl, n-propyl, isopropyl, n-butyl, sec-butyl, tert-butyl, n-pentyl, neopentyl, n-hexyl, 2-ethyl-n-hexyl, n-lauryl, cyclohexyl, phenyl and benzyl.

2. 2-Oxo-1,3-dioxolane-4-carboxylic acid ester of Claim 1, selected from 4-methoxycarbonyl-2-oxo-1,3-dioxolane and 4-ethoxycarbonyl-2-oxo-1,3-dioxolane.

3. 2-Oxo-1,3-dioxolane-4-carboxylic acid ester of the general formula (V) as depicted in claim 1, **characterized in that** R₁ is an n-valent radical derived by abstraction of the OH groups of an n-valent polyol and which may be substituted by at most n-1 further 2-oxo-1,3-dioxolane-4-carboxylate groups of the general formula (VI)

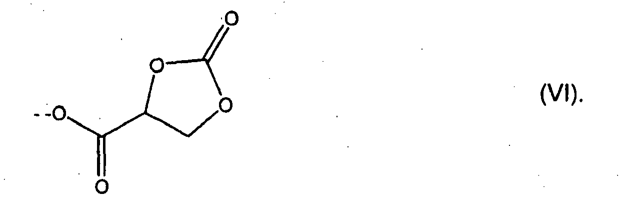

4. 2-Oxo-1,3-dioxolane-4-carboxylic acid ester of Claim 3, **characterized in that** the n-valent polyol comprises C₂₋₄-(poly)oxyalkylene groups.

5. 2-Oxo-1,3-dioxolane-4-carboxylic acid ester of Claim 3 or 4, **characterized in that** n = 2 to 5.

6. Process for the preparation of a 2-oxo-1,3-dioxolane-4-carboxylic acid ester of one of the Claims 3 to 5, **characterized in that** a 2-oxo-1,3-dioxolane-4-carboxylic acid ester according to Claim 1 or 2 is transesterified with an n-valent polyol according to the definition of one of the Claims 3 to 5.

7. Process of Claim 6, **characterized in that** the transesterification is carried out in the presence of an enzymatic catalyst or an acidic cation exchanger.

8. Use of a 2-oxo-1,3-dioxolane-4-carboxylic acid ester of one of the Claims 1 to 5 for the preparation of hydroxyurethanes.

9. Use of a 2-oxo-1,3-dioxolane-4-carboxylic acid ester of Claim 1 or 2 as end group for the blocking of amines.

## Patentansprüche

1. 2-Oxo-1,3-dioxolan-4-carbonsäureester der allgemeinen Formel (V), 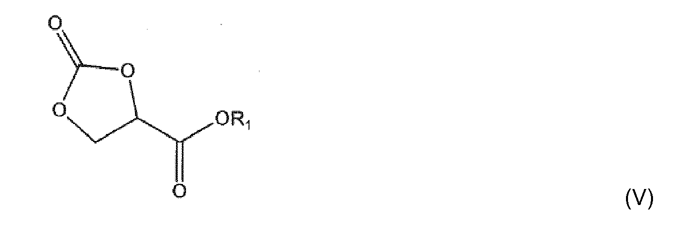 wobei R₁ aus Methyl, Ethyl, n-Propyl, iso-Propyl, n-Butyl, sec-Butyl, tert-Butyl, n-Pentyl, neo-Pentyl, n-Hexyl, 2-Ethyl-n-hexyl, n-Lauryl, Cyclohexyl, Phenyl und Benzyl ausgewählt ist.

2. 2-Oxo-1,3-dioxolan-4-carbonsäureester nach Anspruch 1, ausgewählt aus 4-Methoxycarbonyl-2-oxo-1,3-dioxolan und 4-Ethoxycarbonyl-2-oxo-1,3-dioxolan.

3. 2-Oxo-1,3-dioxolan-4-carbonsäureester der allgemeinen Formel (V) gemäß Anspruch 1, **dadurch gekennzeichnet, dass** R₁ ein durch Abstraktion der OH-Gruppen eines n-wertigen Polyols abgeleiteter n-wertiger Rest ist, der mit maximal n-1 weiteren 2-Oxo-1,3-dioxolan-4-carboxylatgruppen der allgemeinen Formel (VI) 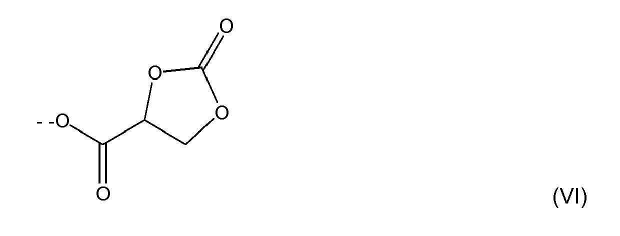 substituiert sein kann.

4. 2-Oxo-1,3-dioxolan-4-carbonsäureester nach Anspruch 3, **dadurch gekennzeichnet, dass** das n-wertige Polyol C₂₋₄-(Poly)Oxyalkylengruppen umfasst.

5. 2-Oxo-1,3-dioxolan-4-carbonsäureester nach Anspruch 3 oder 4, **dadurch gekennzeichnet, dass** n = 2 bis 5 ist.

6. Verfahren zur Herstellung der 2-Oxo-1,3-dioxolan-4-carbonsäureester nach einem der Ansprüche 3 bis 5, **dadurch gekennzeichnet, dass** ein 2-Oxo-1,3-dioxolan-4-carbonsäureester nach Anspruch 1 oder 2 mit einem n-wertigen Polyol gemäß der Definition eines der Ansprüche 3 bis 5 umgeestert wird.

7. Verfahren nach Anspruch 6, **dadurch gekennzeichnet, dass** die Umesterung in Gegenwart eines enzymatischen Katalysators oder eines sauren Kationenaustauschers durchgeführt wird.

8. Verwendung der 2-Oxo-1,3-dioxolan-4-carbonsäureester nach einem der Ansprüche 1 bis 5 zur Herstellung von Hydroxyurethanen.

9. Verwendung der 2-Oxo-1,3-dioxolan-4-carbonsäureester nach Anspruch 1 oder 2 als Endgruppen zum Blockieren von Aminen.

## Revendications

1. Ester d'acide 2-oxo-1,3-dioxolane-4-carboxylique de formule générale (V) 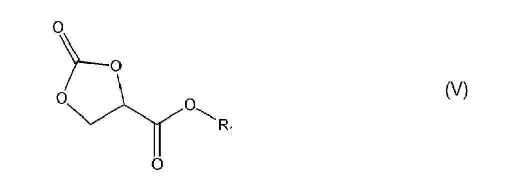 dans laquelle R₁ est choisi parmi méthyle, éthyle, n-propyle, isopropyle, n-butyle, sec-butyle, tertio-butyle, n-pentyle, néopentyle, n-hexyle, 2-éthyl-n-hexyle, n-lauryle, cyclohexyle, phényle et benzyle.

2. Ester d'acide 2-oxo-1,3-dioxolane-4-carboxylique selon la revendication 1, choisi parmi le 4-méthoxy-carbonyl-2-oxo-1,3-dioxolane et le 4-éthoxycarbonyl-2-oxo-1,3-dioxolane.

3. Ester d'acide 2-oxo-1,3-dioxolane-4-carboxylique de formule générale (V) tel qu'illustré selon la revendication 1, **caractérisé en ce que** R₁ est un radical n-valent dérivé par abstraction des groupements OH d'un polyol n-valent et qui peut être substitué par au plus n-1 autres groupements 2-oxo-1,3-dioxolane-4-carboxylate de formule générale (VI)

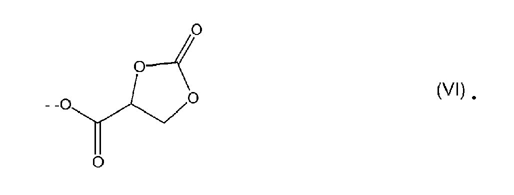

4. Ester d'acide 2-oxo-1,3-dioxolane-4-carboxylique selon la revendication 3, **caractérisé en ce que** le polyol n-valent comprend des groupements C₂₋₄(poly) - oxyalkylène.

5. Ester d'acide 2-oxo-1,3-dioxolane-4-carboxylique selon la revendication 3 ou 4, **caractérisé en ce que** n= 2 à 5.

6. Procédé de préparation d'un ester d'acide 2-oxo-1,3-dioxolane-4-carboxylique selon l'une des revendications 3 à 5, **caractérisé en ce qu'**un ester d'acide 2-oxo-1,3-dioxolane-4-carboxylique selon la revendication 1 ou 2 est transestérifié par un polyol n-valent selon la définition de l'une des revendications 3 à 5.

7. Procédé selon la revendication 6, **caractérisé en ce que** la transestérification est effectuée en présence d'un catalyseur enzymatique ou d'un échangeur de cations acide.

8. Utilisation d'un ester d'acide 2-oxo-1,3-dioxolane-4-carboxylique selon l'une des revendications 1 à 5, pour la préparation d'hydroxyuréthanes.

9. Utilisation d'un ester d'acide 2-oxo-1,3-dioxolane-4-carboxylique selon la revendication 1 ou 2, comme groupement terminal pour le blocage d'amines.
